(19) Europäisches Patentamt
European Patent Office
Office européen des brevets

(11) Publication number: **0 189 203**
**A2**

# (12) EUROPEAN PATENT APPLICATION

(21) Application number: 86100917.3

(22) Date of filing: 23.01.86

(51) Int. Cl.[4]: **C 07 K 5/00**, A 61 K 37/64

(30) Priority: 23.01.85 US 693951
16.01.86 US 818715
16.01.86 US 818734
16.01.86 US 818714

(43) Date of publication of application: 30.07.86
Bulletin 86/31

(84) Designated Contracting States: AT BE CH DE FR GB IT LI LU NL SE

(71) Applicant: ABBOTT LABORATORIES, 14th Street and Sheridan Road North St, North Chicago Illinois 60064 (US)

(72) Inventor: Luly, Jay Richard, 1021 Mayfair, Libertyville, IL (US)
Inventor: Plattner, John Jacob, 1120 Garfield Avenue, Libertyville, IL (US)
Inventor: Rosenberg, Saul Howard, 15 Crestview No. 18, Vernon Hills, IL (US)
Inventor: Kl Fung, Anthony, 4239 Continental, Waukegan, IL (US)

(74) Representative: Modiano, Guido et al, MODIANO, JOSIF, PISANTY & STAUB Modiano & Associati Baaderstrasse 3, D-8000 München 5 (DE)

## (54) Peptidylaminodiols.

(57) The invention relates to renin inhibiting compounds of the formula:

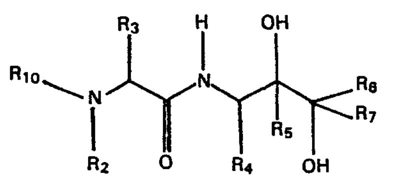

wherein $R_{10}$ is

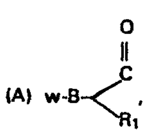

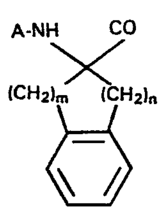

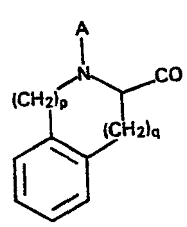

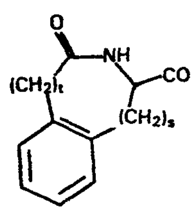

or

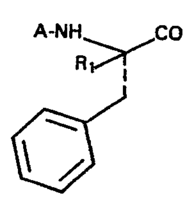

A is hydrogen or an N-protecting group; w is 0 or 1; B is hydrogen, hydroxy, NH, N-alkyl, loweralkyl or arylalkyl; with the proviso that when w is 1, B is NH and when w is 0, B is hydrogen, hydroxy, loweralkyl or arylalkyl; $R_1$ is loweralkyl or lipophilic or aromatic or hydrophilic amino acid side chains; m is 1-3; n is 1-3; p is 1-3; q is 1-3; s is 1-3; t is 0-2; $R_2$ is hydrogen or loweralkyl; $R_3$ and $R_4$ are independently selected from loweralkyl, lipophilic or aromatic amino acid side chains; $R_5$ and $R_7$ are independently selected from hydrogen or loweralkyl; and $R_6$ is hydrogen, loweralkyl, vinyl, arylalkyl or wherein $R_8$ is hydrogen or loweralkyl, X is O, NH or S and $R_9$ is hydrogen, loweralkyl or alkanoyl or $XR_9$ together can be loweralkylsulfonyl, $N_3$ or Cl.

EP 0 189 203 A2

## Technical Field

This is a continuation-in-part of U.S. patent application, Serial No. 693,951, filed January 23, 1985.

The present invention relates to novel organic compounds which inhibit renin, processes for making such compound, synthetic intermediates employed in these processes and method of treating hypertension with such compounds.

## Background Art

Renin is a proteolytic enzyme synthesized and stored principally in a specific part of the kidney called the juxtaglomerular apparatus. Any of three different physiologic circumstances may cause the release of renin into the circulation: (a) a decrease in the blood pressure entering or within the kidney itself; (b) a decrease in the blood volume in the body; or (c) a fall in the concentration of sodium in the distal tubules of the kidney.

When renin is released into the blood from the kidney, the renin-angiotensin system is activated, leading to vasoconstriction and conservation of sodium, both of which result in increased blood pressure. The renin acts on a circulating protein, angiotensinogen, to cleave out a fragment called angiotensin I (AI). AI itself has only slight pharmacologic activity but, after additional cleavage by a second enzyme, angiotensin converting enzyme (ACE), forms the potent molecule angiotensin II (AII). The major pharmacological effects of AII are vasoconstriction and stimulation of the adrenal cortex to release aldosterone, a hormone which causes sodium retention. AII is cleaved by an aminopeptidase to form angiotensin III (AIII), which, compared to AII, is a less potent vasoconstrictor but a more potent inducer of aldosterone release.

Inhibitors of renin have been sought as agents for control of hypertension and as diagnostic agents for identification of cases of hypertension due to renin excess.

With these objectives in mind, the renin-angiotension system has been modulated or manipulated, in the past, with ACE inhibitors. However, ACE acts on several substrates other than angiotensin I (AI), most notably the kinins which cause such undesirable side effects as pain, "leaky" capillaries, prostaglandin release and a variety of behavioral and neurologic effects. Further, ACE inhibition leads to the accumulation of AI. Although AI has much less vasoconstrictor activity than AII, its presence may negate some of the hypotensive effects of the blockade of AII synthesis.

Inhibition of other targets in the renin-angiotensin system such as AII with compounds such as saralasin can block AII activity, but would leave unimpaired and perhaps enhance the hypertensive effects of AIII.

On the other Hand, there are no known side effects which result when renin is inhibited from acting on its substrate. Considerable research efforts have thus been carried out to develop useful inhibitors of renin. Past research efforts have been directed to renin antibodies, pepstatin, phospholipids and substrate analogs such as tetrapeptides and octapeptides to tridecapeptides. These inhibitors either demonstrate poor activity in inhibiting renin production or poor specificity for inhibiting renin only. However, Boger et al. have reported that statine-containing peptides possess potent and specific renin-inhibiting activity (Nature, Vol. 303, p. 81, 1983). In addition, Szelke and co-workers have described polypeptide analogs

containing a non-peptide link (Nature, Vol. 299, p. 555, 1982) which also cause potent renin inhibition and show a high specificity for this enzyme.

Disclosure of the Invention

In accordance with the present invention, there are renin inhibiting compounds of the formula:

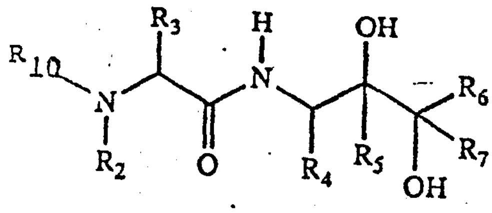

wherein $R_{10}$ is

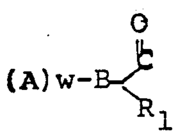, 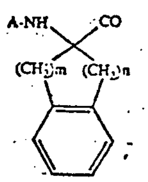; 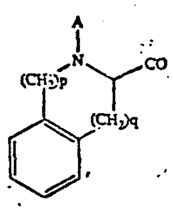, 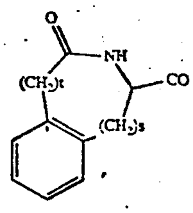, or 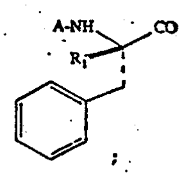;

A is hydrogen or an N-protecting group; w is 0 or 1; B is hydrogen, hydroxy, NH, N-alkyl, loweralkyl or arylalkyl; with the proviso that when w is 1, B is NH and when w is 0, B is hydrogen, hydroxy, loweralkyl or arylalkyl; $R_1$ is loweralkyl or lipophilic or aromatic or hydrophilic amino acid side chains; m is 1-3; n is 1-3; p is 1-3; q is 1-3; s is 1-3; t is 0-2; $R_2$ is hydrogen or loweralkyl; $R_3$ and $R_4$ are independently selected from loweralkyl, lipophilic or aromatic amino acid side chains; $R_5$ and $R_7$ are independently selected from hydrogen or loweralkyl; and $R_6$ is hydrogen, loweralkyl, vinyl, arylalkyl or 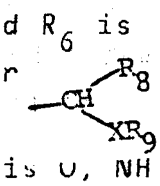

wherein $R_8$ is hydrogen or loweralkyl, X is O, NH or S and $R_9$ is hydrogen, loweralkyl or alkanoyl or $XR_9$ together can be loweralkylsulfonyl, $N_3$ or Cl.

The chiral centers of the compounds of the invention may have either the "R" or "S" configuration but preferably have an "S" configuration except where noted.

The term "N-protecting group" as used herein refers to those groups intended to protect the N-terminus against undesirable reactions during synthetic procedures or to prevent the attack of exopeptidases on the final compounds or to increase the solubility of the final compounds and includes but is not limited to acyl, acetyl, pivaloyl, t-butylacetyl, t-butyloxycarbonyl(Boc), carbobenzyloxycarbonyl or benzoyl groups or an L- or D- aminoacyl residue, which may itself be N-protected similarly.

The term "loweralkyl" as used herein refers to straight or branched chain alkyl radicals containing from 1 to 6 carbon atoms including but not limited to methyl, ethyl, n-propyl, iso-propyl, n-butyl, iso-butyl, sec-butyl, 2-methylhexyl, n-pentyl, 1-methylbutyl, 2,2-dimethylbutyl, 2-methylpentyl, 2,2-dimethylpropyl, n-hexyl and the like.

The term "arylalkyl" as used herein refers to an unsubstituted or substituted aromatic ring appended to an alkyl radical including but not limited to benzyl, 1-and 2-naphthylmethyl, halobenzyl and alkoxybenzyl.

The term "alkanoyl" as used herein refers to an acyl radical which has appended to it an alkyl radical.

The term "cycloalkylalkyl" as used herein refers to an alicyclic residue appended to an alkyl radical and includes but is not limited to cyclohexylmethyl and cyclopentylmethyl.

The term "lipophilic or aromatic amino acid side chains" as used herein refers to those amino acid side chains which have an affinity for lipids or have an aromatic ring and include but are not limited to

isobutyl, isopropyl, sec-butyl, benzyl, imidazole-4-yl-methyl, p-hydroxybenzyl, 1- and 2-naphthylmethyl, and cyclohexylmethyl. The term "hydrophilic amino acid side chains" as used herein refers to those amino acid side chains which have an affinity for water and include but are not limited to hydroxymethyl, hydroxyethyl, hydroxypropyl, hydroxybutyl, aminomethyl, aminoethyl, aminopropyl, and aminobutyl. General reference to amino acid side chains in both the description and claims herein is to be taken as reference to such, whether naturally occurring in proteins or not, and to both D- and L-forms.

The terms "Ala", "His", "Leu", "Phe" "Tyr", and "Cys" as used herein refer to alamine, histidine, leucine, phenylalanine, tyrosine and cysteine, respectively.

The following Examples will serve to further illustrate preparation of the novel compounds of the invention.

Example 1

4(S)-t-Butyloxycarbonylamino-5-cyclohexyl-3(R,S)-hydroxy-1-pentene

To a stirred -78°C solution of Boc-cyclohexylalanine methyl ester (10.2 g, 35.8 mmol) in dry toluene (60 ml) was added diisobutylaluminum hydride (34 ml of a 1.5 M solution in toluene). After 30 min., vinyl magnesium bromide (108 ml of 1 M solution in tetrahydrofuran (THF)) was added. After stirring for 15 hours at 0°C, the mixture was carefully quenched with methanol, treated with Rochelle salts (22 ml of saturated aqueous solution in 140 ml $H_2O$), and filtered. After extracting the solids 5 times with ethyl acetate, the extracts and filtrate were combined and the organic phase was washed with brine, dried,

filtered and evaporated to an oil (10.2 g). Chromatography on silica gel eluting with hexane/ethyl acetate mixtures provided 6.1 g (60%) of the desired product.

Anal. calcd. for $C_{16}H_{29}NO_3 \cdot 1/4H_2O$: C, 66.8; H, 10.3; N, 4.9. Found: C,66.9; H, 10.2; N, 4.7.

Example 2

4(S)-Cyclohexylmethyl-5(R,S)-vinyl-2-oxazolidinone

The resultant product of Example 1 (2.80 g, 9.88 mmol) in dry dimethylformamide (DMF) (50 ml) was added to a stirred suspension of NaH (593 mg of a 60% dispersion in oil, 14.8 mmol, hexane washed) in dry DMF (50 ml). After 3 hours, the mixture was quenched (750 ml water + 100 ml brine) and extracted with ether (5 x 100 ml). The combined organic phase was washed with brine (3 x 50 ml), dried ($MgSO_4$), filtered and evaporated to an oil (2.23 g). The NMR spectrum of the crude product revealed an 82:18 mixture of 5 S:5 R diastereomers. Silica gel chromatography gave 80% recovery of pure diastereomers. 5 S:

Anal. calcd. for $C_{12}H_{19}NO_2$: C, 68.9; H, 9.1; N, 6.7. Found: C, 68.4; H, 9.2; N, 6.5. Mass spectrum: $(M+1)^+$ = 210. 5 R: Mass spectrum: $(M+1)^+$ = 210.

Example 3

(3S,4S)-3-Hydroxy-4-amino-5-cyclohexyl-1-pentene

To the resultant 5S-diasteriomer from Example 2 (2.06 g, 9.84 mmol) in dioxane (180 mL) and water (120 mL) was added barium hydroxide octahydrate (6.24 g, 19.8 mmol). The mixture was refluxed for 18 hours, cooled, filtered, concentrated, taken up in water and extracted with ethyl acetate which was dried over $Na_2SO_4$ and

evaporated to afford 1.64 g (91%) of the desired product, m.p. 59-61°C.

Anal. calcd. for $C_{11}H_{21}NO$: C, 72.08; H, 11.55; N, 7.64. Found: C, 71.67; H, 11.68; N, 7.36.

Example 4

(3S,4S)-3-Hydroxy-4-tert-butoxycarbonylamino-5-cyclohexyl-1-pentene

To the resultant compound from Example 3 (1.62 g, 8.84 mmol) in methylene chloride (20 mL) was added di-tert-butyldicarbonate (1.93 g, 8.84 mmol). The mixture was stirred for 14 hours, diluted with ethyl acetate, washed sequentially with 0.5 M $H_3PO_4$, saturated $NaHCO_3$ solution and brine, then dried over $Na_2SO_4$ and evaporated to afford 2.51 g (100%) of the desired compound.

Example 5

(3S,4S)-3-tert-Butyldimethylsilyloxy-4-tert-butoxycarbonylamino-5-cyclohexyl-1-pentene

To the resultant compound from Example 4 (0.264 g, 0.932 mmol) in DMF (4 mL) was added tert-butyldimethylsilyl chloride (0.300 g, 1.99 mmol) and imidazole (0.269 g, 3.95 mmol). The mixture was stirred at room temperature for 12 hours, poured into ethyl acetate and washed sequentially with 0.5 M $H_3PO_4$, saturated $NaHCO_3$ solution and brine, then dried over $Na_2SO_4$ and evaporated to afford 0.355 g (96%) of the desired compound. Mass spectrum: $(M+H)^+$ = 398.

Example 6

(2RS,3R,4S)-3-tert-Butyldimethylsilyloxy-4-tert-butoxycarbonylamino-5-cyclohexyl-1,2-oxopentane

The resultant compound from Example 5 (0.355 g, 0.893 mmol) in methylene chloride (8 mL) was treated with m-chloroperbenzoic acid (0.758 g, 3.51 mmol) and

stirred at ambient temperature for 14 hours. The mixture was concentrated, dissolved in ethyl acetate, washed sequentially with cold 10% aqueous $Na_2SO_3$ solution, saturated $NaHCO_3$ solution and brine, and then dried over $Na_2SO_4$ and evaporated to afford 0.374 g (100%) of the desired compound. Mass spectrum: $(M+H)^+ = 404$.

Example 7

(2RS,3R,4S)-3-Hydroxy-4-tert-butoxycarbonylamino-5-cyclohexyl-1,2-oxopentane

The resultant compound from Example 6 (2.10 g, 5.07 mmol) was treated with 1 M tetrabutylammonium fluoride in tetrahydrofuran (10 mL). The mixture was stirred at 0°C for 1 hour, poured into ethyl acetate, washed with water and brine, then dried over $Na_2SO_4$ and evaporated. Chromatography on silica gel (0.5% methanol in chloroform) afforded 1.13 g (74%) of the desired compound. Mass spectrum: $(M+H)^+ = 300$.

Example 8

(2S,3R,4S)-1-Azido-2,3-dihydroxy-4-tert-butoxycarbonylamino-5-cyclohexylpentane

The resultant compound from Example 7 (1.12 g, 3.74 mmol), ammonium chloride (0.374 g, 6.98 mmol) and sodium azide (0.580 g, 8.92 mmol) were refluxed in methanol (25 mL) for 12 hours. The mixture was concentrated, then taken up in ethyl acetate, washed with water and brine, dried over $Na_2SO_4$ and evaporated. Chromatography on silica gel (20% ether in hexane) afforded 0.461 g (36%) of the desired compound followed by 0.323 g (25%) of the 4-R isomer. 4S-Diasteriomer: m.p. 93-94°C. 4R-Diasteriomer: mass spectrum: $(M+H)^+ = 343$.

Example 9

(2S,3R,4S)-1-Amino-2,3-dihydroxy-4-tert-butoxycarbonylamino-5-cyclohexylpentane

The resultant compound from Example 8 (107 mg, 0.313 mmol) and 10% palladium on carbon (110 mg) in methanol (10 mL) were stirred under a hydrogen atmosphere for 18 hours. The mixture was filtered and evaporated to afford 94.6 mg (96%) of the desired compound. Mass spectrum: $(M+H)^+$ = 317.

Example 10

(2S,3R,4S)-1-(3-Methylbutylcarbonylamino)-2,3-dihydroxy-4-tert-butyloxycarbonylamino-5-cyclohexylpentane

To the resultant compound from Example 9 (94.6 mg, 0.299 mmol) in methylene chloride (5 mL) at 0°C was added 4-methylpentanoyl chloride (52 µl, 0.37 mmol) and triethylamine (71 µl, 0.51 mmol). The mixture was stirred at 0°C for 90 min, diluted with ethyl acetate, washed sequentially with 0.5 M $H_3PO_4$ solution, 2 M NaOH solution and brine, and then dried over $Na_2SO_4$ and evaporated to afford 0.118 g (95%) of the desired compound, m.p. 179-183°C.

Exact mass calcd. for $C_{22}H_{43}N_2O_5$: 415.3172. Found: 415.3166.

Example 11

(2S,3R,4S)-1-(Isobutylsulfonylamino)-2,3-dihydroxy-4-tert-butoxycarbonylamino-5-cyclohexylpentane

Using the procedure of Example 10 and isobutylsulfonyl chloride rather than 4-methylpentanoyl chloride gave the desired compound. Mass spectrum: $(M+H)^+$ = 437.

Example 12

Boc-Phe-d,l-3-pyrazolylalanine Methyl Ester

To dl-3-pyrazolylalanine methyl ester dihydrochloride (2.05 g, 8.5 mmol) in DMF (10 mL) at

-10°C was added Boc-Phe N-hydroxysuccinimide ester (2.50 g, 6.90 mmol) and N-methylmorpholine (2.8 mL, 25 mmol). The mixture was stirred at -10°C for 1 hour and then at 25°C for 12 hours. The mixture was partitioned between ethyl acetate and saturated $NaHCO_3$ solution, and extracted with ethyl acetate which was washed with water, dried over $Na_2SO_4$ and evaporated to afford 2.75 g (95%) of the desired product.

Anal. calcd. for $C_{21}H_{28}N_4O_5$ $0.25 \cdot H_2O$: C, 59.92; H, 6.82; N, 13.31. Found: C, 59.82; H, 6.75; N, 13.13.

## Example 13

## Boc-Phe-d,1-3-pyrazolylalanine

Boc-Phe-dl-3-pyrazolylalanine methyl ester (0.210 g, 0.505 mmol) in dioxane (1.5 mL) and water (1.0 mL) was treated with lithium hydroxide monohydrate (0.0272 g, 0.648 mmol), stirred at 25°C for 30 minutes and quenched with 0.32 mL 2 M HCl. The mixture was poured into chloroform, washed with water, dried over $Na_2SO_4$ and evaporated to afford 0.184 g (91%) of the desired compound.

Anal. calcd. for $C_{20}H_{26}N_4O_5 \cdot 0.25H_2O$: C, 59.03; H, 6.56; N, 13.77. Found: C, 58.66; H, 6.70; N, 13.65.

## Example 14

## Boc-Phe-His Amide of (2S,3R,4S)-1-(3-Methylbutylcarbonylamino)-2,3-dihydroxy-4-amino-5-cyclohexylpentane

The resultant compound from Example 10 (57.0 mg, 0.138 mmol) was stirred in 4 M HCl/dioxane (1.5 mL) for 1 hour and evaporated. The residue was dissolved in dimethylformamide (0.9 mL) and treated with N-methylmorpholine (33 µl, 0.30 mmol).

To Boc-Phe-His-OH (59.1 mg, 0.147 mmol) and 1-hydroxybenzotriazole (58.0 mg, 0.429 mmol) in dimethylformamide (0.8 mL) at -23°C was added 1-(3-dimethylaminopropyl)-3-ethylcarbodiimide hydrochloride (EDAC, 28.7 mg, 0.150 mmol). After stirring at -23°C for 1 hour, the amine solution was added and the reaction was stirred at -23°C for 2 hours then at room temperature for 12 hours. The mixture was poured into saturated $NaHCO_3$ solution and extracted with ethyl acetate which was washed with water and brine, then dried over $Na_2SO_4$ and evaporated. Chromatography of the residue on silica gel (3% methanol in chloroform) afforded 21.5 mg (22%) of the desired compound, m.p. 194-196°C.

## Example 15

### Boc-Phe-d,l-3-pyrazolylalanine Amide of (2S,3R,4S)-1-(3-Methylbutylcarbonylamino)-2,3-dihydroxy-4-amino-5-cyclohexylpentane

Using the procedure of Example 14 and Boc-Phe-d,l-3-pyrazolylalanine-OH rather than Boc-Phe-His-OH afforded the desired compound.

Anal. calcd. for $C_{37}H_{58}N_6O_7 \cdot 0.5H_2O$: C, 62.78; H, 8.40; N, 11.87. Found: C, 62.51; H, 8.17; N, 11.52.

## Example 16

### Boc-Phe-His Amide of (2S,3R,4S)-1-(Isobutylsulfonylamino)-2,3-dihydroxy-4-amino-5-cyclohexylpentane

Using the procedure of Example 14 with the resultant compound from Example 11 afforded the desired compound, m.p. 94-97°C. Mass spectrum: $(M+H)^+ = 721$.

## Example 17

### Boc-Phe-His Amide of (2S,3R,4S)-1-Azido-2,3-dihydroxy-4-amino-5-cyclohexlpentane

Using the procedure of Example 14 with the resultant compound from Example 8 afforded the desired compound, m.p. 158-163°C. Mass spectrum: $(M+H)^+ = 480$.

## Example 18

### Boc-His Amide of (2S,3R,4S)-1-Azido-2,3-dihydroxy-4-amino-5-cyclohexylpentane

Using the procedure of Example 14 with the resultant compound from Example 8 and using Boc-His-OH rather than Boc-Phe-His-OH afforded the desired compound. Mass spectrum: $(M+H)^+ = 657$.

## Example 19

### Boc-(O-methyl)Tyr-His Amide of (2S,3R,4S)-1-Azido-2,3-dihydroxy-4-amino-5-cyclohexylpentane

Using the procedure of Example 14 with the resultant compound from Example 18 and using Boc-(O-methyl)Tyr-OH rather than Boc-Phe-His-OH afforded the desired compound, m.p. 171-173°C. Mass spectrum: $(M+H)^+ = 561$.

## Example 20

### Boc-Phe-Ala-OH Amide of (2S,3R,4S)-1-Azido-2,3-dihydroxy-4-amino-5-cyclohexylpentane

The resultant compound from Example 8 (53.0 mg, 0.155 mmol) was stirred in 4 M HCl/dioxane for 1 hour and evaporated. The residue was taken up in tetrahydrofuran (3 mL), treated with N-methylmorpholine (18 µl, 0.16 mmol) and cooled to 0°C.

To Boc-Phe-Ala-OH (58.0 mg, 0.172 mmol) in tetrahydrofuran (2 mL) at -12°C was added N-methylmorpholine (19 µl, 0.17 mmol) followed by isobutylchloroformate (22 µl, 0.17 mmol). After 3 minutes the amine solution was added and the mixture was stirred for 15 minutes at -12°C and 2 hours at room temperature. The mixture was diluted with ethyl acetate and washed sequentially with 0.5 M $H_3PO_4$, saturated $NaHCO_3$ solution and brine, then dried over $Na_2SO_4$ and evaporated. Chromatography of the residue on silica

gel (3% methanol in chloroform) afforded 86.8 mg (100%) of the desired compound. Mass spectrum: $(M+H)^+ = 561$.

Example 21

4(S)-Cyclohexylmethyl-5(R)-[1(R,S),2-oxoethyl]-2-oxazolidinone

To a stirred solution of the 5(R) diastereomer of Example 2 (0.40 g, 1.9 mmol) in dichloromethane (13 ml) was added 3-chloroperoxybenzoic acid (500 M%). After 5 days, the mixture was diluted with ether and washed with 1 M $Na_2SO_3$, saturated $K_2CO_3$ and brine. Drying and evaporation provided the desired product (0.34 g, 80%). Mass spectrum: $M^+ = 225$.

Example 22

4(S)-Cyclohexylmethyl-5(R)-[1(R,S)-hydroxy-2-(isopropylmercapto)ethyl]-2-oxazolidinone

To a stirred solution of the resultant product of Example 21 (0.31 g, 1.38 mmol) in methanol were added triethylamine (0.19 ml) and isopropyl mercaptan (105 mg). The mixture was heated at 50-60°C overnight, evaporated and chromatographed on silica gel eluting with ethyl acetate/hexane mixtures to give the 1(R) isomer (110 mg, 27%), the 1(S) isomer (70 mg, 17%) and mixed fractions (110 mg, 27%).

Mass spectrum of 1(R) isomer: $M^+ = 301$.
Mass spectrum of 1(S) isomer: $M^+ = 301$.

Example 23

4(S)-Amino-5-cyclohexylmethyl-2(R,S),3(R)-dihydroxy-1-isopropylmercaptopentane

The 1(R,S) mixture of Example 22 (100 mg, 0.33 mmol) and $Ba(OH)_2 \cdot 8\ H_2O$ (209 mg, 0.66 mmol) were refluxed in 1/1, dioxane/water (8 ml, degassed) for 7 hours. The mixture was then diluted with dioxane,

filtered, evaporated, dissolved in dichloromethane, filtered and evaporated. Silica gel chromatography provided 86 mg (96%) of the desired product.

## Example 24

### Boc-Phe-His Amide of 4(S)-amino-5-cyclohexylmethyl-2(R),3(R)-dihydroxy-1-isopropylmercaptopentane and Boc-Phe-His Amide of 4(S)-amino-5-cyclohexylmethyl-2(S),3(R)-dihydroxy-1-isopropylmercaptopentane

The Boc-Phe-His coupling procedure of Example 14 was used, except the amine hydrochloride and N-methylmorpholine were replaced with the resultant product of Example 23, and EDAC was replaced with 1,3-dicyclohexylcarbodiimide (DDC). Separation was achieved on silica gel (9/1, $CH_2Cl_2/CH_3OH$) to give 20% yield of the desired compounds.

Anal. calcd. for 2(R) isomer ($C_{34}H_{53}N_5O_6S \cdot 3/4H_2O$): C, 60.7; H, 8.2; N, 10.4. Found: C, 60.8; H, 8.2; N, 9.8. FAB mass spectrum: $(M+1)^+$ = 660.

Anal. calcd. for 2(S) isomer ($C_{34}H_{53}N_5O_6S \cdot 1/2H_2O$): C, 61.0; H, 8.1; N, 10.4. Found: C, 60.9; H, 8.3; N, 10.0 FAB mass spectrum: $(M+1)^+$ = 660.

## Example 25

### Boc-Phe-His Amide of 4(S)-amino-5-cyclohexylmethyl-2(R),3(R)-dihydroxy-1-(isopropylsulfonyl)pentane

A solution of the 2(R) isomer from Example 24 (10 mg, 0.015 mmol) in dichloromethane was treated with 3-chloroperoxybenzoic acid (5.2 mg, 200 M%) for 3 hours. The desired compound was isolated in 92% yield after silica gel chromatography (9/1, dichloromethane/methanol). FAB mass spectrum: $(M+1)^+$ = 692.

## Example 26

4(S)-Cyclohexylmethyl-5(R)-[1(R,S)-hydroxy-2-(isopropyloxy)ethyl]-2-oxazolidinone

A solution of the resultant product of Example 21 (0.22 g, 1.0 mmol) in dimethylformamide (DMF, 1.0 mL) was added to a stirred solution of sodium isopropoxide (2.0 mmol) in DMF (9.0 mL). After warming for 24 hours, the mixture was evaporated and then partitioned between 1 M HCl and ethyl acetate. The organic phase was washed aq. $NaHCO_3$ and brine. Drying and evaporating gave the desired alcohols in 62% yield.

## Example 27

Boc-Phe-His Amide of 4(S)-Amino-5-cyclohexylmethyl-2(R),3(R)-dihydroxy-1-isopropyloxypentane and Boc-Phe-His Amide of 4(S)-Amino-5-cyclohexylmethyl-2(S),3(R)-dihydroxy-1-isopropyloxypentane

The 1(R,S) mixture of Example 26 was hydrolyzed according to the procedure of Example 23 to give the desired amines which were coupled to Boc-Phe-His according to the procedure of Example 24. Separation was achieved on silica gel (9/1, $CH_2Cl_2/CH_3OH$) to give 22% yield of the desired compounds.

## Example 28

Ethoxycarbonyl-Leu-Leu Amide of 4(S)-Amino-5-cyclohexyl-3(R,S)-hydroxy-1-pentene

The resultant product of Example 1 (1.05 g, 3.70 mmol) was dissolved in anhydrous 2.2 M $HCl/CH_3OH$ (70 ml). Evaporation after 16 hours gave the corresponding amine hydrochloride.

To a stirred -13°C solution of ethoxycarbonyl-Leu-Leu-OH (1.17 g, 3.70 mmol) in anhydrous tetrahydrofuran (37 mL) were added N-methylmorpholine (NMM, 0.41 mL) and isobutyl chloroformate (0.50 g). After 3 minutes, a -13°C THF

solution of NMM (0.41 mL) and the above amine hydrochloride were added. The mixture was warmed to room temperature for 6 hours and then partitioned between ethyl acetate (60 mL) and 1 M $H_3PO_4$ (15 mL). The organic phase was washed with water, aq. $NaHCO_3$ and brine. Drying and evaporating provided 1.76 g (99%) of the desired product. Mass spectrum: $M^+ = 481$.

Anal. calcd. for $C_{26}H_{47}N_3O_5$: C, 64.8; H, 9.8; N, 8.7. Found: C, 64.6; H, 10.1; N, 8.5

Example 29

Ethoxycarbonyl-Leu-Leu Amide of 4(S)-amino-5-cyclohexyl-3(R,S)-hydroxy-1,2(R,S)-oxopentane

To a stirred solution of the product of Example 28 (350 mg, 0.727 mmol) in dichloromethane (12 mL) was added 3-chloroperoxybenzoic acid. After 48 hours, the solution was partitioned between ether (28 mL) and 1 M $Na_2SO_3$ (3 mL). The layers were separated, and the organic phase was washed with 1 M $Na_2SO_3$, water, saturated $NaHCO_3$ and brine. Drying ($Na_2SO_4$) and evaporating provided 0.341 g of the hydroxy epoxide mixture which was used without further purification.

Example 30

Ethoxycarbonyl-Leu-Leu Amide of 4(S)-Amino-1-chloro-5-cyclohexyl-2(R,S),3(R,S)-dihydroxypentane

To 200 mg (0.402 mmol) of the resultant product of Example 29 was added 4 M HCl in anhydrous dioxane (5 ml). After 1 hour, the homogeneous solution was evaporated *in vacuo*. Chromatography provided the diols in a combined 60% yield.

Example 31

Boc-Phe-Ala Amide (4-amino) of (2S,3R,4S)-1,4-Diamino-2,3-dihydroxy-5-cyclohexylpentane

A solution of the resultant compound of Example 20 (59.3 mg, 0.106 mmol) in methanol (4 ml) was

hydrogenated at atmospheric pressure (10% Pd/C) for 16 hours. Filtration and evaporation provided 49 mg (87%) of the desired compound. Mass spectrum: $(M+H)^+ = 535$.

## Example 32

**Ethoxycarbonyl-Leu-Leu Amide of (2RS,3RS,4S)-Amino-5-cyclohexyl-1,2,3-trihydroxypentane**

To a stirred solution of the resultant compound of Example 28 (200 mg, 0.415 mmol), N-methylmorpholine N-oxide (112 mg), and $OsO_4$ (0.13 ml of a 2.5 w/v % solution in t-butanol) in THF (10 ml) was added water (1 ml). After 16 hours, brine was added, and the mixture was extracted exhaustively with ether. The combined organic phase was washed sequentially with 10% aq. $Na_2SO_3$, 1 M $H_3PO_4$, and brine. The solution was dried ($Na_2SO_4$), filtered, and evaporated to give 205 mg (96%) of the desired product as a glassy solid. Mass spectrum $(M+H)^+ = 516$.

## Example 33

**2(S)-t-Butyloxycarbonylamino-1-cyclohexyl-3(R,S)-hydroxy-6-methyl-4-heptyne**

To a -78°C solution of n-butyl lithium (51.2 ml of a 0.91 M solution in hexane) was added 3-methylbutyne (3.52 g, 51.8 mmol) over the course of 30 seconds. Anhydrous THF (10 ml) was added, and the cold bath was removed for 30 minutes. After cooling the reaction mixture back to -78°C, Boc-cyclohexylalaninal [prepared by Swern oxidation (Mancuso, A.J.; Huang, S.-L.; and Swern, D. J. Org. Chem. 1978, 43, 2480) of 5.3 g, 20.7 mmol of Boc-cyclohexylalaninol] in dry THF (5 ml) was added over 5 minutes. After 1 hour the reaction was quenched by addition of $NH_4Cl$ (4.01 g, 75 mmol) in water (30 ml). The resulting mixture was partitioned between ether (50 ml) and water (30 ml). The organic phase was washed with water (15 ml), saturated $NaHCO_3$,

and brine. Drying and evaporating provided an oil which was chromatographed on $SiO_2$ (ethyl acetate/hexane, 15/85) to give 4.52 g (68%) of the desired product as a 2:1 mixture of hydroxy diastereomers. Mass spectrum: $(M+H)^+$ = 323.

Anal. calcd. for $C_{19}H_{33}NO_3$: C, 70.5; H, 10.3; N, 4.3. Found: C, 70.1; H, 10.6; N, 4.3.

## Example 34

### 2(S)-t-Butyloxycarbonylamino-1-cyclohexyl-3(R,S)-hydroxy-6-methyl-3(Z)-heptene

The resultant product of Example 4 (0.510 g, 1.70 mmol) and quinoline (0.425 ml) in ethyl acetate (20 ml) were hydrogenated over 10% $Pd/BaSO_4$ (31.8 mg) for 5 hours. The mixture was filtered, the catalyst was washed with ethyl acetate, and the combined organic phase was washed sequentially with 1 M HCl (10 ml, 0°C), water (10 ml), saturated $NaHCO_3$, and brine (10 ml). Drying ($Na_2SO_4$) and evaporating in vacuo provided 0.510 g (99%) of the desired product. Mass spectrum: $(M+H)^+$ = 325.

Anal. calcd. for $C_{19}H_{35}NO_3$: C, 70.1; H, 10.8; N, 4.3. Found: C, 70.3; H, 11.2; N, 4.3.

## Example 35

### 2(S)-t-Butyloxycarbonylamino-1-cyclohexyl-6-methyl-3,4,5-trihydroxyheptane

Following the procedure of Example 32, but replacing the resultant product of Example 28 with the resultant compound of Example 34 and increasing the temperature to reflux and the time to 10 days, gave the desired product as a mixture of 4 diastereomers. Chromatography on silica gel provided 3 of them pure eluting in the order shown. Isomer A: mp: 178-179°C; Mass spectrum: $M^+$ = 359.

Anal. calcd. for $C_{19}H_{37}NO_5$: C, 63.5; H, 10.4; N, 3.9. Found: C, 63.4; H, 10.1; N, 3.8. Isomer B: mp 148-149°C; Mass spectrum: $M^+$ = 359; Found: C, 63.3; 10.2; N, 3.8. Isomer C: mp 151-152°C; Mass spectrum: $M^+$ = 359; Found: 63.5; 10.1; N, 3.8.

## Example 36

### Ethoxycarbonyl-Phe-Leu Amide of 2(S)-Amino-1-cyclohexyl-6-methyl-3,4,5-tri-hydroxyheptane; Isomers A, B, and C

Following the procedure of Example 20, but replacing Boc-Phe-Ala with ethoxycarbonyl-Phe-Leu and systematically replacing the resultant compound of Example 8 with isomers A, B, and C from Example 35, gave the desired products.

Isomer A: Mass spectrum: $(M+H)^+$ = 592. Anal. calcd. for $C_{32}H_{53}N_3O_7$: C, 65.0; H, 9.0; N, 7.1. Found: C, 65.0; H, 8.9; N, 6.9. Isomer B: Mass spectrum: $(M+H)^+$ = 592. Isomer C: Mass spectrum: $(M+H)^+$ = 592. Found: C, 65.2; H, 9.1; N, 7.0.

## Example 37

### (4S)-2,8-Dimethyl-4-[(toluenesulfonyl)amino]-5-nonanone

To a stirred -78°C solution of Ts-Leu (15 g, 53 mmol) in dry THF (240 ml) was added n-butyl lithium (57.8 ml of a 0.91 M solution in hexane) followed 15 minutes later by isopentyl magnesium bromide (185 ml of a 0.8 M solution in THF). The mixture was heated at reflux for 3 days, then cooled and poured into 0°C 1 M HCl (500 ml). The layers were separated and the aqueous phase was extracted with ether (3 x 150 ml). The combined organic layers were washed with saturated $NaHCO_3$ (2 x 150 ml) and brine (150 ml). Drying and evaporating provided a residue which was chromatographed on silica gel to give 7.43 g (41%) of the desired product. Mass spectrum: $(M+H)^+$ = 340.

Anal. calcd. for $C_{18}H_{29}NO_3S$: C, 63.7; H, 8.6, N, 4.1. Found: C, 64.0; H, 8.6; N, 4.1.

Example 38

(4S)-2,8-Dimethyl-5-hydroxy-4-[(toluenesulfonyl)-amino]-5-vinylnonane

To a stirred 0°C solution of the resultant compound of Example 37 (79 mg, 0.23 mmol) in dry THF (8 ml) was added vinyl magnesium bromide (1.5 ml of a 1.0 M solution in THF) dropwise. The mixture was warmed (room temperature, 10 hours), quenched (8 ml $H_2O$ + 2 ml brine), acidified with 0.1 M $H_3PO_4$ (pH = 7), and extracted with ether (3 x 4 ml). The combined ether phase was washed (4 ml brine), dried ($Na_2SO_4$), filtered, and evaporated to give 81 mg (95%) of the desired product as a 4:1 mixture of diastereomers.

Example 39

Boc-Phe-Ala Amide of (4S)-Amino-2,8-dimethyl-5-hydroxy-5-vinylnonane, Isomer A

To a solution of the resultant compound of Example 38 (400 mg, 1.09 mmol) in liquid ammonia (80 ml) was added sodium (150 mg, 6.5 mmol). After 6 hours the ammonia was allowed to slowly evaporate under a stream of nitrogen. Benzene (50 ml) and 1:1, ethanol:water (20 ml) were added with stirring. The layers were separated, and the aqueous phase was extracted with ether. The combined organic phase was dried ($Na_2SO_4$), filtered, and evaporated to give 85 mg (37%) of the desired product.

Following the procedure of Example 20, but replacing the amine hydrochloride and N-methylmorpholine with the above resultant product, gave the desired major diastereomer in 35% yield after chromatography. FAB mass spectrum: $(M+K)^+$ = 570.

Anal. calcd. for $C_{30}H_{49}N_3O_5$: % C, 67.8; H, 9.3; N, 7.9. Found: C, 67.7; H, 9.6; N, 7.3.

## Example 40

Boc-Phe-Ala Amide of (4S)-Amino-2,8-dimethyl-5-hydroxy-5-vinylnonane, Isomer B

Scale up of the procedure of Example 38 led to the isolation of the minor diastereomer pure after chromatography. Treatment as in Example 39 provided the desired isomer of the resultant product of Example 39.

## Example 41

Boc-Phe-Ala Amide of 4(S)-Amino-3-isopentyl-6-methyl-1,2,3-trihydroxyheptane, Isomer A

Following the procedure of Example 32, but replacing the resultant compound of Example 28 with the resultant compound of Example 39 gave the desired product.

## Example 42

Boc-Phe-Ala Amide of 4(S)-Amino-3-isopentyl-6-methyl-1,2,3-trihydroxyheptane, Isomer B

Following the procedure of Example 32, but replacing the resultant compound of Example 28 with the resultant compound of Example 40, gave the desired diastereomer of the resultant product of Example 41.

## Example 43

Ethoxycarbonyl-Phe-Leu Amide of 2(S)-Amino-1-cyclohexyl-6-methyl-3,4,5-trihydroxyheptane, Isomers D, E, F, and G

Following the $Na/NH_3$ reduction procedure of Example 39, but replacing the resultant compound of Example 38 with the resultant product of Example 33 gave the corresponding 3(E)-heptene isomer of the resultant compound of Example 34 which was oxidized according to the procedure of Example 35. In this way the four iomeric 2(S)-t-butyloxycarbonyl-amino-1-cyclohexyl-6-methyl-3,4,5- trihydroxyheptanes were isolated,

separated, and converted to the desired products according to the procedure of Example 36.

Example 44

2(S)-t-Butyloxycarbonylamino-1-cyclohexylbut-3-ene

A 0°C solution of potassium hexamethyldisilazide (22.9 mmol in 115 ml of 5:1, tetrahydrofuran (THF): dimethyl sulfoxide (DMSO) was added dropwise to triphenylmethylphosphonium iodide (24.81 mmol). After stirring at 0°C for 1 hour, the solution was cooled to -78°C and a solution of Boc-cyclohexylalaninal [4.90 g, 19.08 mmol, prepared by Swern oxidation (Mancuso, A.J.; Huang,, S.-L.; and Swern, D., J. Org. Chem. 1978, 43, 2480) of Boc-cyclohexylalaninol] in dry THF (95 ml) was added. After stirring at -78°C for 1 hour, the mixture was allowed to warm to room temperature. The reaction mixture was quenched with aqueous ammonium chloride and extracted with ether (2x300 ml). The combined organic phase was washed with 10% HCl (200 ml), saturated $NaHSO_3$ (2x200 ml), $H_2O$ (2x200 ml), saturated $NaHCO_3$ (2x200 ml), and brine (200 ml), dried ($MgSO_4$), filtered, and evaporated. The residue was purified by chromatography (40 μm $SiO_2$; ether:hexane, 15:85) to give the desired compound in 60% yield. Mass spectrum: $(M+H)^+$ = 254.

Example 45

Boc-Phe-Ala Amide of (2S)-Amino-1-cyclohexylbut-3-ene

The resultant compound of Example 44 (310 mg, 1.22 mmol) was dissolved in 1 M anhydrous HCl in anhydrous methanol (35 ml). After 22 hours, the solvent was evaporated to give 230 mg (99%) of the corresponding amine hydrochloride which was used without further purification.

To a stirred -13°C solution of Boc-Phe-Ala (408 mg, 1.21 mmol) in dry THF (8 ml) containing

N-methylmorpholine (122 mg, 1.21 mmol) was added isobutyl chloroformate (165 mg, 1.21 mmol) dropwise. After 3 minutes, a -13°C solution of the above amine hydrochloride (230 mg, 1.21 mmol) in 1:1, THF:DMF (4 ml) containing N-methylmorpholine (122 mg) was added dropwise. The mixture was warmed to room temperature for 2 hours. Evaporation provided a residue which was partitioned between ethyl acetate (30 ml) and 0.1 M $H_3PO_4$ (10 ml). The organic phase was washed with brine (10 ml), saturated $NaHCO_3$ (10 ml), and brine (10 ml). Drying, filtering, evaporating, and chromatographing (55 g $SiO_2$; 95:5, $CH_2C_{12}$:$CH_3OH$) gave the desired compound (462 mg, 81%).

Example 46

Boc-Phe-Ala Amide of 3(S)-Amino-4-cyclohexyl-1,2(R,S)-dihydroxybutane

To a stirred solution of the resultant compound of Example 45 (100 mg, 0.212 mmol) in THF (5 ml) were added $OsO_4$ solution (0.065 ml of a 2.5 W/V% solution in t-butanol) and N-methylmorpholine N-oxide (57 mg, 0.424 mmol) sequentially. After 4.5 hours, brine (10 ml) was added, and the mixture was extracted with ether (4x8 ml). The combined organic phase was washed with 10% $Na_2SO_3$ (3 x 6 ml), 0.1 M H3PO4 (5 ml), and brine (5 ml). Drying, filtering, and evaporating provided the desired product (97 mg, 91%). Mass spectrum: $M^+$ = 505.

Example 47

3(S)-t-Butyloxycarbonylamino-4-cyclohexyl-1,2(R,S)-dihydroxybutane

To a stirred solution of 2(S)-t-butyloxycarbonylamino-1-cyclohexylbut-3-ene (1.00 g, 3.95 mmol) in THF (20 ml) were added $OsO_4$ solution (1.2 ml of a 2.5 W/V% solution in t-butanol) and N-methylmorpholine

N-oxide (1.07 g, 7.90 mmol). After 24 hours, the mixture was partitioned between ether (50 ml) and brine (25 ml). The layers were separated, and the organic phase was extracted with ether (3 x 25 ml). The combined organic phase was washed with 10% $Na_2SO_3$ (4x10 ml), 1.0 M $H_3PO_4$ (2x8 ml), and brine (15 ml). Drying and evaporating provided the desired product as an oil (1.14 g, 100%). 'H NMR shows a 1:1 mixture of diastereomers (NH 4.43 and 4.56 ppm).

## Example 48

### Boc-Phe-His Amides of 3(S)-Amino-4-cyclohexyl-2(R,S)-hydroxy-1-t-butyldimethylsilyloxybutane

The resultant compound of Example 47 (1.10 g, 3.82 mmol) was treated with anhydrous 1M $HCl/CH_3OH$ (80 ml) for 16 hours at which time evaporation and drying provided the corresponding amine hydrochloride (0.85 g, 100%).

To a suspension of the above hydrochloride salt (344 mg, 1.54 mmol) and imidazole (105 mg) in dichloromethane (15 ml) were added triethylamine (156 mg) and t-butyldimethylsilyl chloride (232 mg). The solvent was evaporated after 31 hours, and the residue was then re-dissolved in anhydrous dimethylformamide (DMF, 15 ml). Boc-Phe-His (619 mg) and 1-hydroxybenzotriazole (HOBT, 312 mg) were then added. After cooling the stirred solution to -23°C, 1,3-dicyclohexyl-carbodiimide (DCC, 318 mg) was added. The mixture was warmed to room temperature 3 hours later. After 13 hours the solvent was evaporated in vacuo, and the residue was dissolved in ethyl acetate (40 ml), filtered, washed with saturated $NaHCO_3$ (2x10 ml) and brine (10 ml), and dried ($Na_2SO_4$). Filtration and evaporation provided a residue which was chromatographed on silica gel eluting with dichloromethane/methanol mixtures to give 441 mg (42%)

of the desired product. Mass spectrum: $(M+H)^+ = 686$.

Anal. calcd. for $C_{36}H_{59}N_5O_6Si$: C, 63.0; H, 8.7; N, 10.2. Found: C, 62.8; H, 9.0; N, 9.9.

Example 49

Boc-Phe-His Amides of 3(S)-Amino-4-cyclohexyl-1,2(R)-dihydroxybutane

To a stirred solution of the resultant product of Example 48 (200 mg, 0.291 mmol) in anhydrous THF (5 ml) at 0°C was added tetrabutylammonium fluoride (0.58 ml of a 1 M solution in THF). The solution was warmed to room temperature for 4 hours and then evaporated. The residue was dissolved in chloroform and washed with water (3X) and brine (1X). Drying and evaporating provided a gum which was treated with hot ethyl acetate (8 ml). Cooling and filtration provided 25 mg of the desired material. Mass spectrum: $(M+H)^+ = 572$.

Anal. Calcd for $C_{30}H_{45}N_5O_6 \cdot 1/2H_2O$: C, 62.1; H, 8.0; N, 12.1. Found: C, 62.4; H, 8.2; N, 12.0.

Example 50

(4S)-2,8-Dimethyl-4-[(toluenesulfonyl)amino]-5-nonanone

To a stirred -78°C solution of Ts-Leu (15 g, 53 mmol) in dry THF (240 ml) was added butyl lithium (57.8 ml of a 0.91 M solution in hexane) followed 15 minutes later by isopentyl magnesium bromide (185 ml of a 0.8 M solution in THF). The mixture was heated at reflux for 3 days, then cooled and poured into 0°C 1 M HCl (500 ml). The layers were separated and the aqueous phase was extracted with ether (3x150 ml). The combined organic layers were washed with saturated $NaHCO_3$ (2x150 ml) and brine (150 ml). Drying and evaporating provided a residue which was chromatographed on silica gel to give 7.43 g (41%) of the desired product. Mass spectrum: $(M+H)^+ = 340$.

Anal. calcd. for $C_{18}H_{29}NO_3S$: C, 63.7; H, 8.6; N, 4.1. Found: C, 64.0; H, 8.6; N, 4.1.

Example 51

(4S)-2,8-Dimethyl-5-hydroxy-4-[(toluenesulfonyl)amino]-5-vinylnonane

To a stirred 0°C solution of the resultant compound of Example 50 (79 mg, 0.23 mmol) in dry THF (8 ml) was added vinyl magnesium bromide (1.5 ml of a 1.0 M solution in THF) dropwise. The mixture was warmed (room temperature, 10 hours), quenched (8 ml $H_2O$ + 2 ml brine), acidified with 0.1 M $H_3PO_4$ (pH=7), and extracted with ether (3 x 4 ml). The combined ether phase was washed (4 ml brine), dried ($Na_2SO_4$), filtered, and evaporated to give 81 mg (95%) of the desired product as a 4:1 mixture of diastereomers.

Example 52

Boc-Phe-Ala Amide of (4S)-Amino-2,8-dimethyl-5-hydroxy-5-vinylnonane

To a solution of the resultant compound of Example 51 (400 mg, 1.09 mmol) in liquid ammonia (80 ml) was added sodium (150 mg, 6.5 mmol). After 6 hours the ammonia was allowed to slowly evaporate under a stream of nitrogen. Benzene (50 ml) and 1:1, ethanol:water (20 ml) were added with stirring. The layers were separated, and the aqueous phase was extracted with ether. The combined organic phase was dried ($Na_2SO_4$), filtered, and evaporated to give 85 mg (37%) of the desired product.

Following the procedure of Example 45, but replacing the amine hydrochloride and N-methylmorpholine with the above resultant product, gave the desired major diastereomer in 35% yield after chromatography. FAB mass spectrum: $(M+K)^+ = 570$.

Anal. calcd. for $C_{30}H_{49}N_3O_5$: % C, 67.8; H, 9.3; N, 7.9. Found: C, 67.7; H, 9.6; N, 7.3.

Example 53

Boc-Phe-Ala Amide of (3S)-Amino-2-hydroxy-2-isopentyl-5-methylhexanal

Following the procedure of Example 46 with the resultant compound of Example 52 except replacing N-methylmorpholine N-oxide with aqueous $NaIO_4$ gave the desired compound.

Example 54

Boc-Phe-Ala Amide of 3-Amino-1,2-dihydroxy-2-isopentyl-5-methylhexane

Treatment of the resultant compound of Example 53 with one equivalent of $NaBH_4$ in methanol provided the desired compound after aqueous work-up.

Example 55

Boc-Phe-Ala Amide of 3-Amino-1,2-dihydroxy-2-isopentyl-5-methylhexane

Scale up of the procedure of Example 51 led to the isolation of the minor diastereomer pure after chromatography. Treatment as in Examples 52, 53 and 54 provided the desired isomer of the resultant product of Example 54.

Example 56

2(S)-t-Butyloxycarbonylamino-1-cyclohexyl-6-methylhept-3-ene

To a stirred -78°C solution of Boc-cyclohexylalanine methyl ester (40 g, 140 mmol) in anhydrous toluene (250 ml) was added diisobutylaluminum hydride (130 M%, 1.5 M solution in toluene, 121.4 ml) at a rate to keep the internal temperature below -60°C. After stirring for an additional 20 minutes at -78°C, the aldehyde solution is used immediately as described below.

To a potassium hydride (35% dispersion in oil, 32.09 g) suspension in a 0°C mixture of anhydrous

THF/DMSO (1000 ml/200 ml) under dry $N_2$ was added 1,1,1,3,3,3-hexamethyldisilazane (209 M%, 49.07 g) dropwise. After stirring at 0°C for 1 hour, the resulting solution was added via cannula to a 0°C flask containing isopentyltriphenylphosphonium bromide (209 M%, 125.66 g). The mixture was stirred vigorously for 1 hour at which time it was cooled to -78°C. The -78°C aldehyde solution prepared above was then added via cannula. After stirring at -78°C for 15 minutes, the mixture was allowed to slowly warm to room temperature and then heated to 40°C for 12 hours. The mixture was then cooled to room temperature and quenched with methanol (7.65 ml) followed by aqueous Rochelle salts (100 ml saturated solution and 500 ml $H_2O$). The mixture was then extracted with ethyl acetate (2x). The combined extracts were washed with water and brine. Drying ($MgSO_4$) and evaporating provided crude alkene which was chromatographed on silica gel (ether/hexane) to give 16.5 g (38%) of the desired compound as an 85:15 mixture of cis:trans isomers. Mp=53-55°C. Mass spectrum: $M^+$ =309.

Anal. calcd. for $C_{19}H_{35}NO_2$: C, 73.7; H, 11.4; N, 4.5. Found: C, 73.8; H, 11.4; N, 4.5.

## Example 57

### 2(S)-t-Butyloxycarbonylamino-1-cyclohexyl-3,4-dihydroxy-6-methylheptane: The 3(R)4(S), 3(S)4(S), 3(R)4(R), and 3(S)4(R) Diastereomers

To a solution of the resultant compound of Example 56 (8.50, 27.5 mmol) in dry THF (150 ml) were added $OsO_4$ (2.8 ml of a 2.5% solution in t-butanol and N-methylmorpholine N-oxide (9.28 g, 68.7 mmol). After 4 days the mixture was partitioned between ether (200 ml) and brine (100 ml). The aqueous layer was back-extracted with ether (2x100 ml), and the combined

organic phase was washed with 10% $Na_2SO_3$, 0.1 M $H_3PO_4$, and brine. Drying ($MgSO_4$) and evaporating provided a residue (10.81 g) which was chromatographed on silica gel to elute a 60% yield of the 4 diols in the following order.

3(R),4(S) Mass spectrum: $(M+H)^+$ = 344. Anal. calcd. for $C_{19}H_{37}NO_4$: C, 66.4; H, 10.9; N, 4.1. Found: C, 66.4; H, 10.8; N, 3.9.

3(S),4(S) Mass spectrum: $(M+H)^+$ = 344. Anal. calcd. for $C_{19}H_{37}NO_4$: C, 66.4; H, 10.9; N, 5.1. Found: C, 66.4; H, 11.1; N, 4.0.

3(R),4(R) Mass spectrum: $(M+H)^+$ = 344.

3(S),4(R) Mass spectrum: $(M+H)^+$ = 344. Anal. calcd. for $C_{19}H_{37}NO_4$: C, 66.4; H, 10.9; N, 4.1. Found: C, 66.0; H, 10.7; N, 4.0.

## Example 58

### Boc-Phe-His Amide of 2(S)-Amino-1-cyclohexyl-3(R),4(S)-dihydroxy-6-methylheptane

The 3(R),4(S) diastereomer of Example 57 was deprotected with HCl/methanol, and the resulting product was coupled to Boc-Phe-His using 1-hydroxybenzotriazole and 1,3-dicyclohexylcarbodiimide according to the procedure of Example 48. The desired product was obtained in 40-60% yield. Mass spectrum: $(M+H)^+$ = 628.

Anal. calcd. for $C_{34}H_{53}N_5O_6 \cdot H_2O$: C, 63.2; H, 8.6; N, 10.8. Found: C, 63.2; H, 8.4; N, 10.5.

## Example 59

### Boc-Phe-His Amide of 2(S)-Amino-1-cyclohexyl-3(S),4(S)-dihydroxy-6-methylheptane

Following the procedure of Example 58, but replacing the 3(R),4(S) diastereomer with the 3(S),4(S) diastereomer gave the desired compound. Mass spectrum: $(M+H)^+$ = 628.

Anal. calcd. for $C_{34}H_{53}N_5O_6 \cdot 1/2H_2O$: C, 64.1; H, 8.6; N, 11.0. Found: C, 64.0; H. 8.6; N, 10.6.

Example 60

Boc-Phe-His Amide of 2(S)-Amino-1-cyclohexyl-3(R),4(R)-dihydroxy-6-methylheptane

Following the procedure of Example 58, but replacing the 3(R),4(S) diastereomer with the 3(R),4(R) diastereomer gave the desired compound. Mass spectrum: $(M+H)^+$ = 628.

Anal. calcd. for $C_{34}H_{53}N_5O_6 \cdot H_2O$: C, 63.2; H, 8.6; N, 10.8. Found: C, 63.1; H, 8.5; N, 10.7.

Example 61

Boc-Phe-His Amide of 2(S)-Amino-1-cyclohexyl-3(S),4(R)-dihydroxy-6-methylheptane

Following the procedure of Example 58, but replacing the 3(R),4(S) diastereomer with the 3(S),4(R) diastereomer gave the desired compound. Mass spectrum: $(M+H)^+$= 628.

Anal. calcd. for $C_{34}H_{53}N_5O_6 \cdot 3/4H_2O$: C, 63.7; H, 8.6; N, 10.9. Found: C, 63.8; H, 8.8; N, 10.7.

Example 62

A. 4(S)-t-Butyloxycarbonylamino-5-cyclohexyl-3(R,S)-hydroxy-1-pentene

To a stirred -78°C solution of Boc-cyclohexylalanine methyl ester (10.2 g, 35.8 mmol) in dry toluene (60 ml) was added diisobutylaluminum hydride (34 ml of a 1.5 M solution in toluene). After 30 minutes, vinyl magnesium bromide (108 ml of 1 M solution in THF) was added. After stirring for 15 hours at 0°C, the mixture was carefully quenched with methanol, treated with Rochelle salts (22 ml of saturated aqueous solution in 140 ml $H_2O$), and filtered. After extracting the solids 5 times with

ethyl acetate, the extracts and filtrate were combined and the organic phase was washed with brine, dried, filtered, and evaporated to an oil (10.2 g). Chromatography on silica gel eluting with hexane/ethyl acetate mixtures provided 6.1 g (60%) of the desired product.

Anal. calcd. for $C_{16}H_{29}NO_3 \cdot 1/4H_2O$: C, 66.8; H, 10.3; N, 4.9. Found: C, 66.9; H, 10.2; N, 4.7.

B. 4(S)-Cyclohexylmethyl-5(R,S)-vinyl-2-oxazolidinone

The resultant product of Example 62A (2.80 g, 9.88 mmol) in dry dimethylformamide (DMF) (50 ml) was added to a stirred suspension of NaH (593 mg of a 60% dispersion in oil, 14.8 mmol, hexane washed) in dry DMF (50 ml). After 3 hours, the mixture was quenched (750 ml water + 100 ml brine) and extracted with ether (5x100 ml). The combined organic phase was washed with brine (3x50 ml), dried ($MgSO_4$), filtered, and evaporated to an oil 2.23 g. The NMR spectrum of the crude product revealed an 82:18 mixture of 5 S:5 R diastereomers. Silica gel chromatography gave 80% recovery of pure diastereomers. 5 S:

Anal. calcd. for $C_{12}H_{19}NO_2$: C, 68.9; H, 9.1; N, 6.7. Found: 68.4; H, 9.2; N, 6.5. Mass spectrum: $(M+1)^+ = 210$. 5 R: Mass spectrum: $(M+1)^+ = 210$.

C. 5(R)-Carboxy-4(S)-cyclohexylmethyl-2-oxazolidinone

To a solution of the compound from Example 62B (1 g, 4.78 mmol) dissolved in 16 ml of benzene and 3 ml of acetic acid was added a solution of 3.01 g of potassium permanganate in 16 ml of water. The resultant two-phase mixture was vigorously stirred and treated by portionwise addition with 153 mg of tetrabutylammonium bromide. After stirring for 2 hours at room temperature, the mixture was quenched with aqueous

sodium bisulfite, acidified to pH=3, and extracted with ethyl acetate. Drying and evaporating gave the desired product as an oil in 59% yield.

D. 4(S)-Cyclohexylmethyl-5(R)-[3-(3-hydroxypentyl)]-2-oxazolidinone

To a solution of the compound from Example 62C dissolved in tetrahydrofuran and cooled to -78°C was added 3.5 equivalents of ethyl magnesium bromide. After stirring at -78°C for 1.5 hours and at room temperature for 1 hour, the reaction mixture was quenched with water and extracted with ether. The dried ethereal extract was evaporated to afford a 73% yield of product.

E. 2(S)-Amino-1-cyclohexyl-3(R)-3,4-dihydroxy-4-ethylhexane

A solution of the compound from Example 62D (1.69 mmol) and barium hydroxide octahydrate (3.38 mmol) in dioxane (60 ml) and water (40 ml) was heated at reflux under $N_2$ for 21 hours. The solid barium carbonate was filtered and the filtrate was partially evaporated. The residue was diluted with water and the resulting solution was extracted with ether. The organic extract was washed with brine solution, dried over $MgSO_4$, and evaporated to give the desired product in 76% yield.

F. Boc-Phe-His Amide of 2(S)-Amino-1-cyclohexyl-3(R)-3,4-dihydroxy-4-ethylhexane

The resultant product of Example 62E was coupled to Boc-Phe-His using 1-hydroxybenzotriazole and 1,3-dicyclohexylcarbodiimide according to the procedure of Example 48 to give the desired product in 55% yield.

Example 63

Boc-His Amide of 2(S)-Amino-1-cyclohexyl-3(R),4(S)-dihydroxy-6-methylheptane

The procedure of Example 58 was followed except Boc-Phe-His was replaced with Boc-His. Mass spectrum: $(M)^+ = 480$.

Anal. calcd. for $C_{25}H_{44}N_4O_5 \cdot 3/4H_2O$: C, 60.8; H, 9.1; N, 11.3. Found: C, 60.9; H, 9.2; N, 11.0.

Example 64

TBA-CHA-His Amide of 2(S)-Amino-1-cyclohexyl-3(R),4(S)-dihydroxy-6-methylheptane

The resultant compound of Example 63 was deprotected with HCl/methanol, and the resulting product was coupled to t-butylacetyl-cyclohexylalanine (TBA-CHA) using the DCC/HOBT method of Example 48.

Example 65

Ethoxycarbonyl-($OCH_3$)Tyr-His Amide of 2(S)-Amino-1-cyclohexyl-3(R),4(S)-dihydroxy-6-methylheptane

Using the procedure of Example 64, but replacing TBA-CHA with ethoxycarbonyl-(OCH3)Tyr-His gave the desired compound. Mass spectrum: $(M+H)^+$ = 630.

Example 66

Acetyl-N-methylPhe-His Amide of 2(S)-Amino-1-cyclohexyl-3(R),4(S)-dihydroxy-6-methylheptane

Using the procedure of Example 64, but replacing TBA-CHA with acetyl-N-methylPhe gave the desired compound. Mass spectrum: $M^+$ = 583.

Example 67

Ac-Pl-His Amide of 2(S)-Amino-1-cyclohexyl-3(R),4(S)-dihydroxy-6-methylheptane

Using the procedure of Example 64, but replacing TBA-CHA with O-acetyl-L-3-phenyllactic acid (Ac-Pl-OH) gave the desired compound.

Example 68

Pl-His Amide of 2(S)-Amino-1-cyclohexyl-3(R),4(S)-dihydroxy-6-methylheptane

The resultant compound of Example 67 (37.4 mg, 0.065 mmol) in MeOH at 0°C was treated with $K_2CO_3$ (9.1 mg, 0.065 mmol) for 30 minutes at 0°C. Evaporation

provided a residue which was partitioned between ethyl acetate and water. The organic phase was washed (brine), dried ($MgSO_4$), and evaporated to give the desired compound (32 mg, 93%). Mass spectrum: $(M+H)^+$ = 529.

Anal. calcd. for $C_{31}H_{46}N_4O_6 \cdot 1/2H_2O$: C, 64.8; H, 8.4; N, 10.4. Found: C, 64.6; H, 8.3; N, 10.1.

Example 69

Boc-$\alpha$-Nal-His Amide of 2(S)-Amino-1-cyclohexyl-3(R),4(S)-dihydroxy-6-methyl-heptane

Using the procedure of Example 64, but replacing TBA-CHA with Boc-$_{\alpha}$-naphthylalanine (Boc-$\alpha$-Nal) provided the desired compound. Mass spectrum: $(M+H)^+$ = 678.

Example 70

Dba-His Amide of 2(S)-Amino-1-cyclohexyl-3(R),4(S)-dihydroxy-6-methylheptane

Using the procedure of Example 64, but replacing TBA-CHA with 2,2-dibenzylacetic acid (Dba-OH) gave the desired compound.

Example 71

Pp-His Amide of 2(S)-Amino-1-cyclohexyl-3(R),4(S)-dihydroxy-6-methylheptane

Using the procedure of Example 64, but replacing TBA-CHA with 3-phenyl-propionic acid (Pp-OH) gave the desired compound. Mass spectrum: $(M+H)^+$ = 513.

Anal. calcd. for $C_{29}H_{44}N_4O_4 \cdot 1/2\ H_2O$: C, 66.8; H, 8.7., N, 10.7. Found: C, 66.6; H, 8.8; N, 10.5.

## Example 72

Ethoxycarbonyl-Phe-His Amide of 2(S)-Amino-1-cyclohexyl-3(R),4(S)-dihydroxy-6-ethylheptane

Using the procedure of Example 64, but replacing TBA-CHA with ethoxycarbonyl-Phe gave the desired product. Mass spectrum: $(M+H)^+ = 600$.

Anal. calcd. for $C_{32}H_{49}N_5O_6 \cdot 1/2H_2O$: C, 63.1; H, 8.3; N, 11.5. Found: C, 62.8; H, 8.3; N, 11.4

## Example 73

Ac-Phe-His Amide of 2(S)-Amino-1-cyclohexyl-3(R),4(S)-dihydroxy-6-methylheptane

Using the procedure of Example 64, but replacing TBA-CHA with acetyl-Phe gave the desired product. Mass spectrum: $(M+H)^+ = 570$.

Anal. calcd. for $C_{31}H_{47}N_5O_5 \cdot 1/2H_2O$: C, 64.3; H, 8.2; N, 12.1. Found: C, 64.2; H, 8.3; N, 12.0.

## Example 74

Boc-Leu-His Amide of 2(S)-Amino-1-cyclohexyl-3(R),4(S)-dihydroxy-6-methylheptane

Using the procedure of Example 64, but replacing TBA-CHA with Boc-Leu gave the desired product. Mass spectrum: $(M+H)^+ = 594$.

Anal. calcd. for $C_{31}H_{55}N_5O_6 \cdot 1/2H_2O$: C, 61.8; H, 9.4; N, 11.6. Found: C, 61.8; H, 9.3; N, 11.6.

## Example 75

Tbac-Phe-His Amide of 2(S)-Amino-1-cyclohexyl-3(R),4(S)-dihydroxy-6-methylheptane

Using the procedure of Example 64, but replacing TBA-CHA with t-butyl-aminocarbonyl-Phe

(Tbac-Phe) gave the desired product. Exact mass calcd for $C_{34}H_{55}N_6O_5$: 627.4233. Found: 627.4226.

Example 76

Boc-Phe-Ala Amide of 2(S)-Amino-1-cyclohexyl-3(R),4(S)-dihydroxy-6-methylheptane

Using the procedure of 45, but replacing the resultant compound of Example 44 with the 3(R),4(S) diastereomer of Example 57 gave the desired compound. Mass spectrum: $(M-H)^+ = 560$.

Anal. calcd. for $C_{31}H_{51}N_3O_6$: C, 66.3; H, 9.1; N, 7.5. Found: C, 66.0; H, 9.2; N, 7.3.

Example 77

Boc-Phe-Phe Amide of 2(S)-Amino-1-cyclohexyl-3(R),4(S)-dihydroxy-6-methylheptane

Using the procedure of Example 76, but replacing Boc-Phe-Ala with Boc-Phe-Phe, gave the desired product. Mass spectrum: $(M+H)^+ = 638$.

Anal. calcd. for $C_{37}H_{55}N_3O_6$: C, 69.7; H, 8.7; N, 6.6. Found C, 69.4; H, 8.8; N, 6.5

Example 78

Boc-Phe-PAla Amide of 2(S)-Amino-1-cyclohexyl-3(R),4(S)-dihydroxy-6-methylheptane

Using the procedure of Example 76, but replacing Boc-Phe-Ala with Boc-Phe-(3-pyrazoyl)alanine (Boc-Phe-PAla), gave the desired compound. Mass spectrum: $(M+H)^+ = 628$.

Anal. calcd. for $C_{34}H_{53}N_5O_6 \cdot 1/2H_2O$: C, 64.1; H, 8.5; N, 11.0. Found: C, 64.1; H, 8.3; N, 11.2.

Example 79

Ethoxycarbonyl-Phe-Leu Amide of 2(S)-Amino-1-cyclohexyl-3(R),4(S)-dihydroxy-6-methylheptane

Using the procedure of Example 76, but replacing Boc-Phe-Ala with Boc-Phe-Leu, gave the desired compound. Mass spectrum: $(M+H)^+ = 576$.

Anal. calcd. for $C_{32}H_{53}N_3O_6$: C, 66.7; H, 9.3; N, 7.3. Found: C, 66.4; H, 9.5; N, 7.2.

## Example 80

### Boc-Phe-($SCH_3$)Cys Amide of 2(S)-Amino-1-cyclohexyl-3(R),4(S)-dihydroxy-6-methylheptane

Using the procedure of Example 76, but replacing Boc-Phe-Ala with Boc-Phe-($SCH_3$)Cys, gave the desired compound. Mass spectrum: $(M+H)^+ = 608$.

Anal. calcd. for $C_{32}H_{53}N_3O_6S$: C, 62.8; H, 8.8; N, 6.9. Found: C, 62.8; H, 8.9; N, 6.6.

## Example 81

### Ts-(N Me,$N_{IM}$Bn)-His Amide of 2(S)-Amino-1-cyclohexyl-3(R),4(S)-dihydroxy-6-methylheptane

Using the procedure of Example 63, but replacing Boc-His with Ts-(N Me,$N_{IM}$Bn)-His (DuVigneau, V.; Behrens, O.K. J. Biol. Chem. 1937, 117, 27), gave the desired compound. Mass spectrum: $(M+H)^+ = 639$.

## Example 82

### Ethoxycarbonyl-Phe-MeHis Amide of 2(S)-Amino-1-cyclohexyl-3(R),4(S)-dihydroxy-6-methylheptane

To a stirred -78°C solution of the resultant compound of Example 81 (100 mg, 0.156 mmol) in liquid $NH_3$ (5 ml) and dry tetrahydrofuran (5 ml) was added sodium until a dark green/brown color persisted for 5 minutes. Solid, powdered $NH_4Cl$ was then added, and the mixture was evaporated. The residue was suspended in water and extracted several times with chloroform. The combined extracts were dried ($Na_2SO_4$), filtered, and evaporated to give the MeHis amide of 2(S)-amino-1-cyclohexyl-3(R),4(S)-dihydroxy-6-methylheptane. The material was coupled to ethoxycarbonyl-Phe using to DCC/HOBT method described in Example 48 to give the desired product. Mass spectrum: $(M+H)^+ = 614$.

Example 83

2(S)-t-Butyloxycarbonylamino-1-cyclohexyl-7-methyloct-3-ene

Using the procedure of Example 56, but replacing isopentyltriphenylphosphonium bromide with isohexyltriphenylphosphonium bromide, gave the desired product.

Example 84

2(S)-t-Butyloxycarbonylamino-1-cyclohexyl-3(R),4(S)-dihydroxy-7-methyloctane

Using the procedure of Example 57, but replacing the resultant compound of Example 56 with the resultant compound of Example 83, gave the desired compound.

Example 85

Boc-His Amide of 2(S)-Amino-1-cyclohexyl-3(R),4(S)-dihydroxy-7-methyloctane

Using the procedure of Example 63, but replacing the 3(R),4(S) diastereomer of Example 57 with the resultant compound of Example 84, gave the desired product. Mass spectrum: $(M+H)^+ = 495$.

Anal. calcd. for $C_{26}H_{46}N_4O_5 \cdot 1/2H_2O$: C, 62.0; H, 9.4; N, 11.1. Found: C, 62.2; H, 9.4; N, 10.9.

Example 86

TBA-Phe-His Amide of 2(S)-Amino-1-cyclohexyl-3(R),4(S)-dihydroxy-7-methyloctane

Using the procedure of Example 58, but replacing the resultant compound of Example 57,and Boc-Phe-His with the resultant compound of Example 85 and t-butylacetyl(TBA)-Phe gave the desired compound. Mass spectrum: $(M+H)^+ = 640$.

Anal. calcd. for $C_{36}H_{57}N_5O_5 \cdot 3/4H_2O$: C, 66.2; H, 9.0; N, 10.7. Found: C, 66.1; H, 9.1; N, 10.6.

## Example 87

### 2(S)-t-Butyloxycarbonylamino-1-cyclohexyl-5-methylhex-3-ene

Using the procedure of Example 56, but replacing isopentyltriphenylphosphonium bromide with isobutyltriphenylphosphonium bromide, gave the desired product. Mass spectrum: $M^+ = 295$.

Anal. calcd. for $C_{18}H_{33}NO_2 \cdot 1/4H_2O$: C, 72.0; H, 11.3; N, 4.7. Found: 71.7; H, 11.1; N, 4.5.

## Example 88

### 2(S)-t-Butyloxycarbonylamino-1-cyclohexyl-3(R),4(S)-dihydroxy-5-methylhexane

Using the procedure of Example 57, but replacing the resultant compound of Example 56 with the resultant compound of Example 87, gave the desired compound.

## Example 89

### Boc-Phe-His Amide of 2(S)-Amino-1-cyclohexyl-3(R),4(S)-dihydroxy-5-methylhexane

Using the procedure of Example 58, but replacing the resultant product of Example 57 with the resultant product of Example 88, gave the desired product. Mass spectrum: $(M+H)^+ = 614$.

## Example 90

### Ethoxycarbonyl-Phe-Leu Amide of 2(S)-Amino-1-cyclohexyl-3(R),4(S)-dihydroxyhexane

Following the procedures used to make the resultant compound of Example 79, but replacing isopentyltriphenylphosphonium bromide with propyltriphenylphosphonium bromide, gave the desired product. Mass spectrum: $M^+ = 547$.

Anal. calcd. for $C_{30}H_{49}N_3O_6 \cdot 1/4H_2O$: C, 65.2; H, 9.0; N, 7.6. Found: C, 65.0; H, 8.9; N, 7.3.

## Example 91

### Ethoxycarbonyl-Phe-Leu Amide of 2(S)-Amino-1-cyclohexyl-3(R),4(S)-dihydroxy-5-phenylpentane

Following the procedures used to make the resultant compound of Example 79, but replacing isopentyltriphenylphosphonium bromide with phenethyltriphenylphosphonium bromide, gave the desired product.

## Example 92

### Boc-Phe-His Amide of 2(S)-Amino-1-cyclohexyl-3(R),4(S)-dihydroxypentane

Following the procedures used to make the resultant compound of Example 58, but replacing isopentyltriphenylphosphonium bromide with ethyltriphenylphosphonium bromide, gave the desired product. Mass spectrum: (M+H)·= 600.

Anal. calcd. for $C_{32}H_{49}N_5O_6 \cdot 1/4H_2O$: C, 63.6; H, 8.3; N, 11.6. Found: C, 63.6; H, 8.3; N, 11.5.

## Example 93

### 2(S)-t-Butyloxycarbonylamino-1-cyclohexyl-3(S)-hydroxyhex-5-ene

To a stirred -78°C solution of Boc-cyclohexylalanine methyl ester (35.0 g, 123 mmol) in anhydrous toluene (200 ml) was added diisobutylaluminum hydride (140 M%, 1.5 M solution in toluene, 117 ml) at a rate to keep the internal temperature below -60°C. After stirring for an additional 20 minutes at -78°C, allyl magnesium chloride (184 ml of a 2.0 M solution in THF) was added. The mixture was allowed to stand at 0°C for 16 hours and was then quenched with methanol. The mixture was diluted with ether and then washed

sequentially with citric acid (aq) and brine. Drying ($MgSO_4$) and evaporating provided an oil which was purified by silica gel chromatography to give the desired compound in 40% yield.

Example 94

2(S)-t-Butyloxycarbonylamino-1-cyclohexyl-3(R),4(S)-dihydroxyhex-5-ene

An allylic oxidation using stoiciometric $SeO_2$ and t-butyl hydroperoxide (Umbriet, M.A. and Sharpless, K.B. J. Am. Chem. Soc. 1977, 99, 5526) was performed on the resultant product of Example 93 to give the desired product after silica gel chromatography.

Example 95

Ethoxycarbonyl-Phe-His Amide of 2(S)-Amino-1-cyclohexyl-3(R),4(S)-dihydroxy-hex-5-ene

Following the procedure of Example 58, but replacing the resultant product of Example 57 and Boc-Phe-His with the resultant product of Example 94 and ethoxycarbonyl-Phe-His, gave the desired product.

Example 96

2(S)-Butyloxycarbonylamino-1-cyclohexyl-6-methylhept-3-ene

To a stirred -78°C solution of Boc-cyclohexylalanine methyl ester (40 g, 140 mmol) in anhydrous toluene (250 ml) was added diisobutylaluminum hydride (130 M%, 1.5 M solution in toluene, 121.4 ml) at a rate to keep the internal temperature below -60°C. After stirring for an additional 20 minutes at -78°C, the aldehyde solution is used immediately as described below.

To a potassium hydride (35% dispersion in oil, 32.09 g) suspension in a 0°C mixture of anhydrous tetrahydrofuran/dimethyl sulfoxide (THF/DMSO) (1000

ml/200 ml) under dry $N_2$ was added 1,1,1,3,3,3-hexamethyldisilazane (209 M%, 49.07 g) dropwise. After stirring at 0°C for 1 hour, the resulting solution was added via cannula to a 0°C flask containing isopentyltriphenylphosphonium bromide (209 M%, 125.66 g). The mixture was stirred vigorously for 1 hour at which time it was cooled to -78°C. The -78°C aldehyde solution prepared above was then added via cannula. After stirring at -78°C for 15 minutes, the mixture was allowed to slowly warm to room temperature and then heated to 40°C for 12 hours. The mixture was then cooled to room temperature and quenched with methanol (7.65 ml) followed by aqueous Rochelle salts (100 ml saturated solution and 500 ml $H_2O$). The mixture was then extracted with ethyl acetate (2x). The combined extracts were washed with water and brine. Drying ($MgSO_4$) and evaporating provided crude alkene which was chromatographed on silica gel (ether/hexane) to give 16.5 (38%) of the desired compound as an 85:15 mixture of cis:trans isomers. Mp = 53-55°C. Mass spectrum: $M^+$ = 309.

Anal. calcd. for $C_{19}H_{35}NO_2$: C, 73.7; H, 11.4; N, 4.5. Found: C, 73.8; H, 11.4; N, 4.5.

## Example 97

### 2(S)-t-Butyloxycarbonylamino-1-cyclohexyl-3,4-dihydroxy-6-methylheptane: The 3(R)4(S), 3(S)4(S), 3(R)4(R), and 3(S)4(R) Diastereomers

To a solution of the resultant compound of Example 96 (8.50, 27.5 mmol) in dry THF (150 ml) were added $OsO_4$ (2.8 ml of a 2.5% solution in t-butanol) and N-methylmorpholine N-oxide (9.28 g, 68.7 mmol). After 4 days the mixture was partitioned between ether (200 ml) and brine (100 ml). The aqueous layer was back-extracted with ether (2 x 100 ml), and the combined

organic phase was washed with 10% $Na_2SO_3$, 0.1 M $H_3PO_4$, and brine. Drying ($MgSO_4$) and evaporating provided a residue (10.81 g) which was chromatographed on silica gel to elute a 60% yield of the 4 diols in the following order.

3(R),4(S) Mass spectrum: $(M+H)^+$ = 344.Anal. calcd. for $C_{19}H_{37}NO_4$: C, 66.4; H, 10.9; N, 4.1. Found: C, 66.4; H, 10.8; N, 3.9.

3(S),4(S) Mass spectrum: $(M+H)^+$ = 344. Anal. calcd. for $C_{19}H_{37}NO_4$: C, 66.4; H, 10.9; N, 4.1. Found: C, 66.4; H, 11.1; N, 4.0.

3(R),4(R) Mass spectrum: $(M+H)^+$ = 344.

3(S),4(R) Mass spectrum: $(M+H)^+$ = 344. Anal. calcd. for $C_{19}H_{37}NO_4$: C, 66.4; H, 10.9; N, 4.1. Found: C, 66.0; H, 10.7; N, 4.0.

## Example 98

### Boc-His Amide of 2(S)-Amino-1-cyclohexyl-3(R),4(S)-dihydroxy-6-methylheptane

The 3(R),4(S) diastereomer of Example 97 (1.26 g, 3.67 mmol) was treated with 2.3 M $HCl/CH_3OH$ (32 ml, anhydrous) for 16 hours at which time evaporation and vacuum drying provided the corresponding amine hydrochloride (1.01 g, 98%).

To a stirred -20°C solution of the above salt (0.60 g, 2.1 mmol), Boc-His (0.548 g), 1-hydroxybenzotriazole (HOBT, 0.43 g), and N-methylmorpholine (0.239 g) was added 1,3-dicyclohexylcarbodiimide (DCC, 0.442 g). The mixture was warmed to room temperature over 3 hours and then stirred for an additional 18 hours. The mixture was diluted with ethyl acetate and washed with saturated aq. $NaHCO_3$ and brine. Drying and evaporating provided a solid which was recrystallized to give the desired compound (0.51 g, 50%, 2 crops). Mass spectrum: $M^+$ = 480.

Anal. calcd. for $C_{25}H_{44}N_4O_5 \cdot 3/4H_2O$: C, 60.8; H, 9.1; N, 11.3. Found: C, 60.9; H, 9.2; N, 11.0.

Example 99

2-Ethoxycarbonylaminoindan-2-carboxylic Acid (EtOC-AICA)

To a stirred refluxing mixture of 2-aminoindan-2-carboxylic acid (1.90 g, 10.7 mmol, Pinder, R.M.; Butcher, B.H.; Buxton, D.A.; and Howells, O.J. J. Med. Chem. 1971, 14, 892) in ethyl acetate (200 ml) was added ethyl chloroformate (1.16 g, 10.7 mmol). After 15 hours, the mixture was cooled, filtered, evaporated, and vacuum dried to give 1.17 g (44%) of a white solid.

Example 100

EtOC-AICA-His Amide of 2(S)-Amino-1-cyclohexyl-3(R),4(S)-dihydroxy-6-methylheptane

The resultant compound of Example 98 (50 mg, 0.104 mmol) was deprotected with 2.2 M $HCl/CH_3OH$ (6 ml, anhydrous). Evaporation after 6 hours provided the corresponding amine dihydrochloride.

To a stirred -12°C solution of the resultant compound of Example 99 (25.9 mg, 0.104 mmol, EtOC-AICA-OH) and N-methylmorpholine (10.5 mg) in dry THF was added isobutylchloroformate (14.4 mg). After 3 minutes, the above salt and N-methylmorpholine (21 mg) in dry DMF was added. The mixture was warmed to room temperature for 3 hours, diluted with ethyl acetate and washed sequentially with brine, saturated. aq. $NaHCO_3$, and brine. Drying and evaporating provided a residue which was chromatographed on silica gel eluting with dichloromethane/methanol mixtures to give 30 mg (48%) of the desired compound. Mass spectrum: $(M+H)^+ = 612$.

## Example 101

### N-Carboxy-2-aminoindan-2-carboxylic Acid Anhydride

The resultant compound of Example 99 (500 mg, 2.00 mmol) was dissolved in thionyl chloride (3 ml). Evaporation after 17 hours provided a solid which was recrystallized from ethyl acetate/hexane to give 239 mg (59%) of the desired product.

## Example 102

### AICA-His Amide of 2(S)-Amino-1-cyclohexyl-3(R),4(S)-dihydroxy-6-methylheptane

To a stirred 0°C solution of the amine dihydrochloride prepared in Example 100 (0.104 mmol) and triethylamine (21.0 mg) in dry DMF (5 ml) was added a solution of the resultant compound of Example 101 (21.1 mg, 0.104 mmol) in DMF (0.5 ml). After 16 hours, the mixture was diluted with saturated. aq. $NaHCO_3$ (10 ml) and extracted with ethyl acetate several times. The combined organic phase was washed (brine), dried ($Na_2SO_4$), filtered, and evaporated to a solid which was purified by silica gel chromatography (dichloromethane/methanol) to give 28 mg (50%) of the desired compound. Mass spectrum: (M+H) = 540.

Anal. calcd. for $C_{30}H_{45}N_5O_4$: C, 66.8; H, 8.4; N, 13.0. Found: C, 67.1; H, 8.5; N, 13.0.

## Example 103

### 2(R,S)-Amino-1,2,3,4,-tetrahydronaphthalene-2-carboxylic Acid, (R,S-ATCA)

A stirred suspension of 7,8-benzo-1,3-diazaspiro[4.5]decane-2,4-dione (1.73 g, 8.00 mmol) and $Ba(OH)_2 \cdot 8 H_2O$ in water (12 ml) was heated in a sealed tube at 190°C for 2 hours. The suspension was then filtered while warm. The solids were washed with

water, and the combined aqueous phase was treated with ammonium carbonate. Filtration, evaporation, re-solution, and lyopholization provided 0.44 g (29%) of the corresponding amino acid. Mass spectrum: $(M+H)^+$ = 192.

Example 104

2(R,S)-Ethoxycarbonylamino-1,2,3,4-tetrahydronaphthalene-2-carboxylic Acid (EtOC-(R,S)-ATCA)

To a stirred 0°C solution of the resultant compound of Example 103 (250 mg, 1.31 mmol) in 1 M NaOH/1 M $NaHCO_3$ (1.31 ml/2.62 ml) was added ethoxycarbonyl- O-hydroxysuccinimide ester (245 mg, 1.31 mmol) in dioxane (2.6 ml). After 22 hours, the mixture was acidified (2 M HCl) and extracted (ethyl acetate, 3 x 10 ml). The combined organic phase was washed (water 3x, brine 2x), dried ($NaSO_4$), filtered, and evaporated to a residue which was chromatographed on silica gel (dichloromethane, methanol) to give 183 mg (53%) of the desired compound. Mass spectrum: $M^+$ = 263.

Anal. calcd. for $C_{14}H_{17}NO_4$: C, 63.8; H, 6.5; N, 5.3. Found: C, 63.4; H, 6.4., N, 5.5.

Example 105

EtOC-(R,S)-ATCA-His Amide of 2(S)-Amino-1-cyclohexyl-3(R),4(S)-dihydroxy-6-methylheptane

Following the procedure of Example 100, but replacing the resultant product of Example 99 with the resultant product of Example 104, gave the desired product. Mass spectrum: $(M+H)^+$ = 626.

Example 106

(R,S)-ATCA-His Amide of 2(S)-Amino-1-cyclohexyl-3(R),4(S)-dihydroxy-6-methylheptane

Using the procedure of Example 101, but replacing the resultant product of Example 99 with the resultant product of Example 104, gave the corresponding

anhydride derivative. Following the procedure of Example 102, but replacing the anhydride derivative of Example 101 with the above anhydride derivative, gave the desired product.

Example 107

2-Ethoxycarbonyl-1,2,3,4-tetrahydro-3(R,S)-isoquinolinecarboxylic Acid (EtOC-TIC)

Following the procedure of Example 104, but replacing the resultant compound of Example 103 with 1,2,3,4-tetrahydro-3-(R,S)-isoquinolinecarboxylic acid hydrochloride, gave the desired product.

Example 108

EtOC-TIC-His Amide of 2(S)-Amino-1-cyclohexyl-3(R),4(S)-dihydroxy-6-methylheptane

Following the procedure of Example 100, but replacing the resultant compound of Example 99 with the resultant compound of Example 107, gave the desired product. Mass spectrum: $(M+H)^+ = 612$.

Example 109

1-Oxo-1,2,3,4-tetrahydro-3(R,S)-isoquinolinecarboxylic Acid (OTIC-OH)

To a solution of sodio diethyl acetamidomalonate (0.046 mol) in ethanol (125 ml) was added o-cyanobenzyl bromide (9 g, 0.046 mol) all at once. The reaction was stirred overnight at room temperature and then distributed between ether and aqueous NaCl. Drying and evaporating gave a solid product. A 1.5 g sample of this material was heated at reflux for 3 hours with 25 ml of 48% HBr. After cooling, the mixture was diluted with aqueous NaCl and was extracted with ethyl acetate. The organic extract was washed, dried, and evaporated to give 250 mg of product, mp 235-238°C. Anal. calcd. for $C_{10}H_9NO_3$: C, 62.82; H, 4.74; N, 7.33. Found: C, 61.69; H, 4.78; N, 7.11.

Example 110

OTIC-His Amide of 2(S)-Amino-1-cyclohexyl-3(R),4(S)-dihydroxy-6-methylheptane

Following the procedure of Example 100, but replacing the resultant compound of Example 99 with the resultant compound of Example 109, gave the desired compound. Mass spectrum: $(M+H)^+ = 554$.

Anal. calcd. for $C_{30}H_{43}N_5O_5 \cdot 1/2H_2O$: C, 64.0; H, 7.9; N, 12.4. Found: C, 63.7; H, 8.4; N, 11.8.

Example 111

2(S)-Ethoxycarbonylamino-2-methyl-3-phenylpropionic Acid (EtOC-AMPA-OH)

Following the procedure of Example 104, but replacing the resultant compound of Example 103 with 2(S)-amino-2-methyl-3-phenylpropionic acid, gave the desired compound. Mass spectrum: $M^+ = 251$.

Example 112

AMPA-His Amide of 2(S)-Amino-1-cyclohexyl-3(R),4(S)-dihydroxy-6-methylheptane

Using the procedure of Example 101, but replacing the resultant product of Example 99 with the resultant product of Example 111, gave the corresponding anhydride derivative. Following the procedure of Example 102, but replacing the anhydride derivative of Example 101 with the above anhydride derivative, gave the desired product. Mass spectrum: $(M+H)^+ = 541$.

Example 113

4(S)-Benzyl-2,4-dimethyloxazol-5-one

2(S)-Acetylamino-2-methyl-3-propionic acid (0.68 g) was refluxed in acetic anhydride (5 ml) for 5

hours. Evaporation under high vacuum gave the desired product in quantitative yield which was used in the next step without further purification.

## Example 114

### Acetyl-AMPA-His Amide of 2(S)-Amino-1-cyclohexyl-3(R),4(S)-dihydroxy-6-methylheptane

Following the procedure of Example 102, but replacing the resultant compound of Example 101 with the resultant compound of Example 113, gave the desired product. Mass spectrum: $(M+H)^+ = 584$.

## Example 115

### N-Carboxy-2-amino-2,2-dibenzylacetic Acid Anhydride

Following the procedure of Example 101, but replacing the resultant compound of Example 99 with 2-benzyloxycarbonylamino-2,2-dibenzylacetic acid (Cbz-ADBA-OH), gave the desired compound. Mass spectrum: $M^+ = 281$.

## Example 116

### ADBA-His Amide of 2(S)-Amino-1-cyclohexyl-3(R),4(S)-dihydroxy-6-methylheptane

Using the procedure of Example 101, but replacing the resultant product of Example 99 with the resultant product of Example 115, gave the corresponding anhydride derivative. Following the procedure of Example 102, but replacing the anhydride derivative of Example 101 with the above anhydride derivative, gave the desired product.

The compounds of the present invention can be used in the form of salts derived from inorganic or organic acids. These salts include but are not limited to the following: acetate, adipate, alginate,

aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptonate, hexanoate, hydrochloride, hydrobromide, hydroiodide, 2-hydroxy-ethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl., and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

The novel compounds of the present invention possess an excellent degree of activity and specificity in treating renin-associated hypertension in a host. The ability of the compounds of the invention to inhibit human renal renin can be demonstrated _in vitro_ by reacting a selected compound at varied concentrations with human renal renin, free from acid proteolytic activity, and with human renin substrate (angiotensinogen) at 37°C and pH 6.0. At the end of the incubation, the amount of angiotensin I formed is measured by radioimmunoassay and the molar concentration required to cause 50% inhibition, expressed as the $IC_{50}$, is calculated. When tested in accordance with the foregoing procedure, the compounds of the invention demonstrated $IC_{50}$'s in the range of $10^{-5}$ to $10^{-10}$.

Total daily dose administered to a host in single or divided doses may be in amounts, for example, from 0.001 to 10 mg/kg body weight daily and more usually 0.01 to 1 mg. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. The term parenteral as used herein includes subcutaneous injections, intravenous, intramuscular, intrasternal injection, or infusion techniques.

Injectable preparation, for example, sterile injectable aqueous or oleagenous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water,

Ringer s solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

The foregoing is merely illustrative of the invention and is not intended to limit the invention to the disclosed compounds. Variations and changes which

are obvious to one skilled in the art are intended to be within the scope and nature of the invention which are defined in the appended claims.

CLAIMS

1. A renin inhibiting compound of the formula

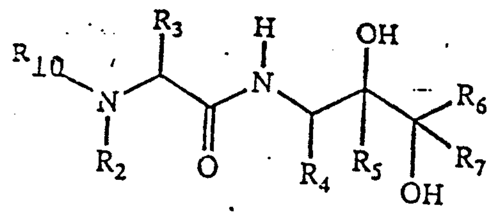

wherein $R_{10}$ is

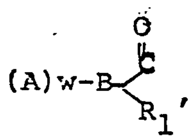
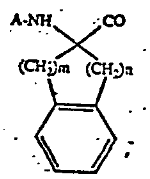
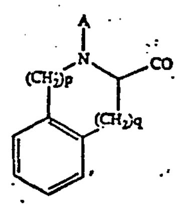
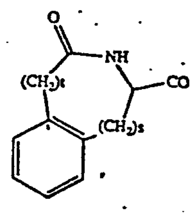
or
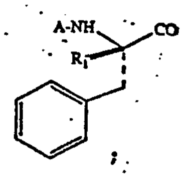

A is hydrogen or an N-protecting group; w is O or 1; B is hydrogen, hydroxy, NH, N-alkyl, loweralkyl or arylalkyl; with the proviso that when w is 1, B is NH and when w is O, B is hydrogen, hydroxy, loweralkyl or arylalkyl; $R_1$ is loweralkyl or lipophilic or aromatic or hydrophilic amino acid side chains; m is 1-3; n is 1-3; p is 1-3; q is 1-3; s is 1-3; t is 0-2; $R_2$ is hydrogen or loweralkyl; $R_3$ and $R_4$ are independently selected from loweralkyl, lipophilic or aromatic amino acid side chains; $R_5$ and $R_7$ are independently selected from hydrogen or loweralkyl; and $R_6$ is hydrogen, loweralkyl, vinyl, arylalkyl or 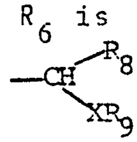

wherein $R_8$ is hydrogen or loweralkyl, X is O, NH or S and $R_9$ is hydrogen, loweralkyl or alkanoyl or $XR_9$ together can be loweralkylsulfonyl, $N_3$ or Cl and pharmaceutically acceptable salts thereof.

2. The renin inhibiting compounds of Claim 1 wherein $R_{10}$ is

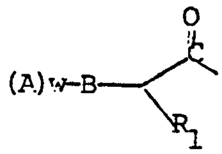

;

$R_7$ is hydrogen; and $R_6$ is 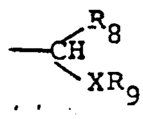 .

3. The renin inhibiting compound of Claim 2 wherein w is 1; B is NH, $R_1$ is benzyl, 4-methoxybenzyl or isobutyl; $R_2$ is hydrogen; $R_3$ is loweralkyl, (4-imidazoyl)methyl or (3-pyrazoyl)methyl; $R_4$ is loweralkyl or cycloalkylmethyl; and $R_5$ is hydrogen.

4. The renin inhibiting compounds of Claim 1 wherein $R_{10}$ is

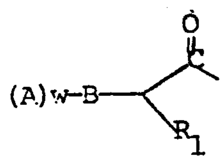

;

and $R_6$ is hydrogen, loweralkyl, vinyl or arylalkyl.

5. The renin inhibiting compounds of Claim 4 wherein A is t-butyloxycarbonyl, t-butylacetyl or ethoxycarbonyl; w is 1; B is NH; $R_1$ is benzyl, cyclohexylmethyl, (4-methox)ybenzyl, (1-naphthyl)methyl or isobutyl; $R_2$ is hydrogen or methyl; $R_3$ is methyl, (4-imidazoyl)methyl, benzyl, (3-pyrazoyl)methyl, (methylmercapto)methyl or isobutyl; $R_4$ is isobutyl or cyclohexylmethyl; $R_5$ is hydrogen or isopentyl; $R_6$ is hydrogen, loweralkyl, vinyl or benzyl; and $R_7$ is hydrogen or ethyl.

6. The renin inhibiting compounds of Claim 5 wherein A is ethoxycarbonyl; $R_1$ is (4-methoxy)benzyl; $R_2$ is hydrogen; $R_3$ is (4-imidazoyl)methyl; $R_4$ is cyclohexylmethyl; $R_5$ and $R_7$ are hydrogen; and $R_6$ is isobutyl.

7. The renin inhibiting compounds of Claim 5 wherein A is ethoxycarbonyl; $R_1$ is benzyl; $R_2$, $R_5$ and $R_7$ are hydrogen; $R_3$ is (4-imidazoyl)methyl; $R_4$ is cyclohexylmethyl; and $R_6$ is isobutyl.

8. The renin inhibiting compounds of Claim 5 wherein A is ethoxycarbonyl; $R_1$ is benzyl; $R_2$, $R_5$ and $R_7$ are hydrogen; $R_3$ and $R_6$ are isobutyl and $R_4$ is cyclohexylmethyl.

9. The renin inhibiting compounds of Claim 1 wherein $R_{10}$ is

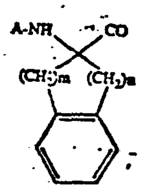 ; 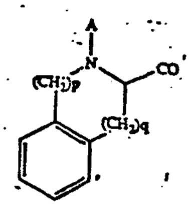 , 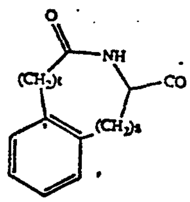 , or 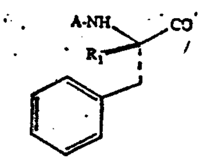 ;

$R_4$ is cyclohexylmethyl; $R_5$ and $R_7$ are hydrogen; and $R_6$ is loweralkyl.

10. The renin inhibiting compound of Claim 9 wherein $R_{10}$ is

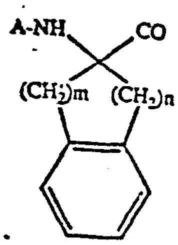 ;

A is ethoxycarbonyl; n is 1; and m is 2.

11. The renin inhibiting compound of Claim 9 wherein $R_{10}$ is

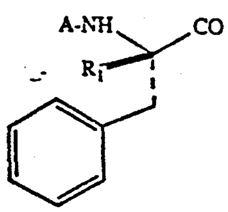 ;

A is hydrogen; and $R_1$ is methyl.

12. A pharmaceutical composition for treating renin-associated hypertension, comprising a pharmaceutical carrier and a therapeutically effective amount of the compound of Claim 1.

13. A method of treating hypertension comprising administering to a host in need of such treatment a therapeutically effective amount of a compound of Claim 1.